# EUROPEAN PATENT SPECIFICATION

(11) **EP 3 474 829 B1**
(45) Date of publication and mention of the grant of the patent: **01.09.2021**
(21) Application number: 16907478.8
(22) Date of filing: 28.06.2016
(51) Int. Cl.: A61K 9/70, A41B 9/12, A61F 13/472, A61F 13/551, A61L 15/20, A61F 13/84, A61L 15/42, A61L 15/34

(54) **INTIMATE SKIN CONDITIONER VEIL COMPRISING SKIN WELL-BEING AGENT**
HAUTPFLEGESCHLEIER FÜR DIE INTIMPFLEGE MIT HAUTSCHUTZMITTEL
VOILE INTIME ADOUCISSANT COMPRENANT UN AGENT DE SOIN DE LA PEAU.

(43) Date of publication of application: 01.05.2019
(73) Proprietor: Essity Hygiene and Health Aktiebolag, 405 03 Göteborg (SE)
(72) Inventor: HÖRLE, Magdalena, 405 03 Göteborg (SE); ALENIUS, Karin, 405 03 Göteborg (SE)
(74) Representative: Nederlandsch Octrooibureau
(86) International application number: PCT/SE2016/050654
(87) International publication number: WO 2018/004405

(56) References cited:
- WO-A1-2006/014693
- WO-A1-2008/147264
- WO-A1-2009/025699
- US-A1- 2006 206 077
- US-A1- 2009 062 753
- US-A1- 2009 062 753
- US-A1- 2009 155 325
- US-A1- 2009 181 073
- US-A1- 2009 181 073

## Description

### TECHNICAL FIELD

The present disclosure relates to an intimate skin conditioner veil configured to be positioned in an undergarment so that it covers the V-zone of a female user, i. e covering the mons pubis in front of the anterior labia commissure of a female user, without covering any part of the vulval vestibule area of the user.

### BACKGROUND ART

Shaving or removing hair from the genital area is an essential part of daily beauty care for today's women. However, removing hair from the genital area of a female user may leave the skin in that area swollen, dry and often irritated. Crèmes and lotions can be used to relieve skin irritation, but may stain the undergarment of the user. The absence of hair in the genital area after hair removal may also lead to the garments of the user chafing the skin and to undesirable skin problems when the skin becomes moist due to the undergarment lying too closely against the skin. US 2009/0181073 describes a method for relieving skin irritation caused by hair removal that includes the application of cold combined with a dry milk product to irritated skin after the hair removal procedure.

### SUMMARY OF THE DISCLOSURE

The present disclosure aims at providing a product, which can avoid or mitigate the problems that may arise after hair removal in the V-zone, said V-zone being a part of the genital area of a female user comprised of mons pubis in front of the anterior labia commissure in the genital area of a female user. The present disclosure thus relates to an intimate skin conditioner veil, which is configured to be positioned in an undergarment so that it covers the mons pubis in front of the anterior labia commissure of the female user, without covering any part of the vulval vestibule area of the user, wherein the veil has a longitudinal central line extending in the longitudinal direction of the veil, and a first extension in the longitudinal direction, between a front end and a rear end of the veil, and a second extension in a transverse direction perpendicular to the longitudinal central line, and wherein the veil comprises a front edge and first and second side edges. The veil comprises a main portion having a length L1 in the longitudinal direction of the veil. The veil has a total longitudinal length L2. The length L1 of the main portion is at least 50% of the total longitudinal length L2 of the veil, wherein the main portion is bounded by a straight front transversal line between front transition points and the first and second side edges, and tapers towards the rear end of the veil, the main portion being reflection symmetric about the longitudinal central line. The veil has a garment facing side and a skin contacting side, the garment facing side and the skin contacting side facing away from one another. The veil further comprises a garment facing layer arranged on the garment facing side of the veil, and a space creating layer arranged on the skin contacting side of the veil. The veil further comprises an additive composition in the form of a skin well-being agent or a carrier substance carrying a skin well-being agent.

The additive composition suitably comprises one or more skin softening agent, or hydrating agent, or cooling agent, or pH regulating agent, or nourishing agent or replenishing agent, or combinations thereof, and may further comprise a skin softening agent in the form of an occluding fat, such as, but not limited to, petrolatum (e.g., VASELINE®).

The additive composition may suitably comprise skin softening agent in the form of moisture binding substances, such as, but not limited to, pidolic acid, urea, lactic acid, glycerol or propylene glycol, and may further comprise a cooling agent, such as, but not limited to, ketals, carboxamides, cyclohexyl derivatives, cyclohexanol derivatives, menthol derivatives, camphor, borneol, eucalyptol, methyl salicylate, tea tree oil, eucalyptus oil.

As said above the skin well-being agent may be carried by a carrier substance, and the carrier substance may suitably include, but is not limited to, propylene glycol, water, dimethicone, oil, petrolatum (e.g., VASELINE®) or alcohol, or combinations thereof.

The skin well-being agent and any carrier substance is preferably present in the intimate skin conditioner veil in a combined amount of 1-300 wt-%, preferably 1-150 wt-%, more preferably 1-50 wt-%, based on the total weight of the intimate skin conditioner veil.

When the skin well-being agent is carried by polyethylene glycol as a carrier substance, the combined amount of skin well-being agent and polyethylene glycol in the intimate skin conditioner veil is preferably 1-100 wt-%, based on the total weight of the intimate skin conditioner veil, more preferably 5-30 wt-%.

The skin well-being agent is advantageously located on the skin contacting side of the intimate skin conditioner veil, and is suitably comprised in a loft layer or a loft portion of the space creating layer.

The skin well-being agent is suitably present in a pattern on the top sheet of the intimate skin conditioner veil.

The intimate skin conditioner veil may preferably further comprise a rear edge which extends substantially in the transverse direction of the veil, at the rear end of the veil, whereby the first and second side edges extend between the front transition points and rear transition points, and the main portion extends in the longitudinal direction between the front transversal line and a straight rear transversal line between the rear transition points, wherein the straight rear transversal line is perpendicular to the longitudinal central line. The main portion may preferably have a front transversal width W1 and a rear transversal width W2, wherein the ratio W1/W2 is 1.1-6.6, preferably 1.5-4.2, more preferably 2.8-3.5. The ratio of the longitudinal lengths L1/L2 is preferably 0.5-1, more preferably 0.6-0.9, most preferably 0.75-0.85. The longitudinal length L1 of the main portion is preferably 60-170 mm, and the total longitudinal length L2 of the veil is preferably 60-220 mm, more preferably 80-150 mm, most preferably 90-120 mm. The main portion preferably has a front transversal width W1 of 100-240 mm, more preferably 140-210 mm, most preferably 155-175 mm and a rear transversal width W2 of 25-95 mm, more preferably 40- 80 mm, most preferably 55-70 mm. The main portion advantageously is tapered such that a straight line between the front transition point and the rear transition point, is inclined in relation to the rear transversal line at an angle v¹, which is greater than 90°, preferably 95-160°, more preferably 100-130°, most preferably 105-125°, and that an angle v² between said straight line and the front transversal line is v² =180° - v¹.

The first and second side edges may advantageously have a curved concave shape between the front and rear transition points. In addition, the main portion may comprise a front main portion located between the front transversal line and a straight intermediate transversal line, and a rear main portion located between the rear transversal line and the intermediate transversal line, wherein longitudinal length of the front main portion is the same as the longitudinal length of the rear main portion, and wherein the curved concave shape of the first and second side edges changes curvature at an intermediate transition point, but runs in the same direction of curvature, wherein the intermediate transition point is located in the rear main portion between the intermediate transversal line and the rear transversal line. The main portion preferably has an intermediate transversal width of 65-90 mm at the intermediate transversal line. Preferably, the first and second side edges changes curvature such that a straight line between the rear transition point and the intermediate transition point, is inclined in relation to the rear transversal line at an angle α, which is > 90°, and a straight line between the front transition point and the intermediate transition point is inclined in relation to the front transversal line at angle β, which is < 180° -α. The angle α is preferably smaller than the angle v¹ between the rear transversal line and the straight line between the rear transition point and the front transition point, and the angle β is preferably smaller than the angle v² between the straight line and the front transversal line.

The intimate skin conditioner veil should preferably have a Gurley stiffness of 5-300 mgf, more preferably 5-100 mgf. The space creating layer may preferably be a combined layer, comprising a loft layer and a top sheet, the loft layer being positioned between the garment facing layer and the top sheet. The loft layer of the space creating layer may suitably have a basis weight of 15-100 gsm and a thickness of 0.5-3.5 mm. Alternatively, the space creating layer may be a multilayer layer comprising a loft portion and a top sheet portion, the loft portion being positioned between the garment facing layer and the top sheet portion. The top sheet is suitably a nonwoven material comprising natural and/or synthetic fibres, and preferably has a basis weight of 8-100 gsm. The garment facing layer is suitably a breathable back sheet, preferably comprising a breathable plastic film or a nonwoven material, such as combinations of spunbond-meltblown layers. The intimate skin conditioner veil should preferably have a moisture vapour transmission rate greater than 1000 g/m²/24h, preferably 1300-1600 g/m²/24h. The thickness of the intimate skin conditioner veil is preferably 0.6-3.8 mm.

Fastening means may suitably be positioned on the garment facing side of the veil, preferably in the form of one or more adhesive or friction areas. The loft layer may be smaller than the garment facing layer and the top sheet. The layers comprised in the veil may suitably be connected by adhesive or ultrasound welded joints.

The intimate skin conditioner veil may further comprising a head portion extending between the front straight line and the front edge, which head portion may comprise at least one protuberance, suitably having a convex shape. The head portion may comprise at least one recess, suitably having a concave shape, and suitably being positioned symmetrically about the longitudinal central line. At least one protuberance may be located on each side of the recess. The intimate skin conditioner veil may also comprise a tail portion extending between the rear transversal line and the rear edge. The tail portion may comprise at least one protuberance and/or recess.

The intimate skin conditioner veil advantageously comprises an additive composition in the form of a skin well-being agent or a carrier substance carrying a skin well-being agent. The additive composition is comprised within the space creating layer.

### BRIEF DESCRIPTION OF THE DRAWINGS

Other features and advantages disclosed herein will become more apparent from the following detailed description of illustrative embodiments when read in conjunction with the attached drawings, wherein:
Fig. 1 shows an illustrative embodiment of a veil placed in a panty;
Fig. 2 shows a cross section of the female anatomy of a wearer and an illustrative embodiment of a veil covering the mons pubis in front of the anterior labia commissure of the wearer;
Fig. 3 is a schematic top view of an illustrative embodiment of an intimate skin conditioner veil;
Fig. 4 schematically illustrates the outer contour of another illustrative embodiment of an intimate skin conditioner veil;
Fig. 5 is a schematic top view of an illustrative embodiment of another intimate skin conditioner veil;
Fig. 6 shows an enlarged portion of the intimate skin conditioner veil shown in Fig. 5;
Fig. 7 is a schematic cross sectional view of an illustrative embodiment of an intimate skin conditioner veil;
Fig. 8 is a schematic cross sectional view of a multilayer space creating layer used in an illustrative embodiment of an intimate skin conditioner veil; and
Fig. 9 schematically illustrates an illustrative embodiment of an intimate skin conditioner veil in which the loft layer is smaller than the other layers;
Figs. 10a-c schematically illustrates illustrative embodiments of packages, in which a wrapping material is wrapped around the intimate skin conditioner veil and sealed along one or more edges.

### DETAILED DESCRIPTION

The present disclosure relates to a product in the form of an intimate skin conditioner veil, which is configured to be positioned in an undergarment so that it covers the mons pubis in front of the anterior labia commissure of a female user, without covering any part of the vulval vestibule area of the user. The part of the female genital area comprised of the mons pubis in front of the anterior labia commissure of a female user, is referred to as the "V-zone".

In an illustrative embodiment, the intimate skin conditioner veil is configured to be positioned in an undergarment so that it contacts the mons pubis in front of the anterior labia commissure of a female user, without contacting any part of the vulval vestibule area of the user.

Accordingly, the intimate skin conditioner veil is essentially different from common hygiene products, which are intended to protect the garments of the wearer, typically by absorbing various body fluids excreted by the wearer. One purpose of the present intimate skin conditioner veil is to protect the skin in the genital area while reducing the risk of absorption of vaginal fluids besides body perspiration. The veil is therefore substantially non-absorbent. By substantially non-absorbent it is meant that the veil can absorb 0.9% by weight sodium chloride solution in an amount of 0-2 times its own weight. For example, to be substantially non-absorbent the veil can be formed from any single material or combination of materials, which include but are not limited to materials composed of a nonwoven material, e g spunbonded, meltblown, carded, hydroentangled, wetlaid, materials essentially consisting of non-absorbent fibers, i.e. at least 95 % of the fibers are non-absorbent fibers, such as at least 99%, or at least 100% of the fibers in the material are non-absorbent fibers. Non-absorbent fibers in the fibrous can be selected from polyolefins, polyesters, polyamides and blends and combinations thereof.

The hair in the genital area acts as a shield against external mechanical stress, and creates an air gap between the skin and the undergarment that contributes to some airiness. When most or all of the hair has been removed, the undergarment material tends to lie more closely against the skin and there is a greater risk that the area becomes moist, which may increase the risk of bacterial growth and odour problem. Skin that has been shaved can also be negatively affected by the shaving itself due to mechanical irritation of the hair follicles, which may lead to an increased risk of infections in the hair follicles. Skin irritation can also be caused by mechanical stress on the skin when the user is moving, as the garment material lies very close to the skin.

The intimate skin conditioner veil serves to act as a protective barrier between the skin and the undergarment material, and to create a space between the skin and the undergarment material. The veil has a garment facing side and a skin contacting side, the garment facing side and the skin contacting side facing away from one another. The veil comprises a garment facing layer arranged on the garment facing side of the veil, and a space creating layer arranged on the skin contacting side of the veil. The veil is configured to be positioned in the undergarment, and therefore preferably comprises fastening means positioned on the garment facing side, most preferably in the form of one or more adhesive or friction areas. For example, adhesive or friction areas can be formed from any single material or combination of materials, which include but are not limited to hooks, friction adhesives, clips, pressure sensitive fastening adhesive. The veil is preferably not attached to the skin of the wearer, and it is therefore preferably essentially free from any adhesive on the skin contacting side.

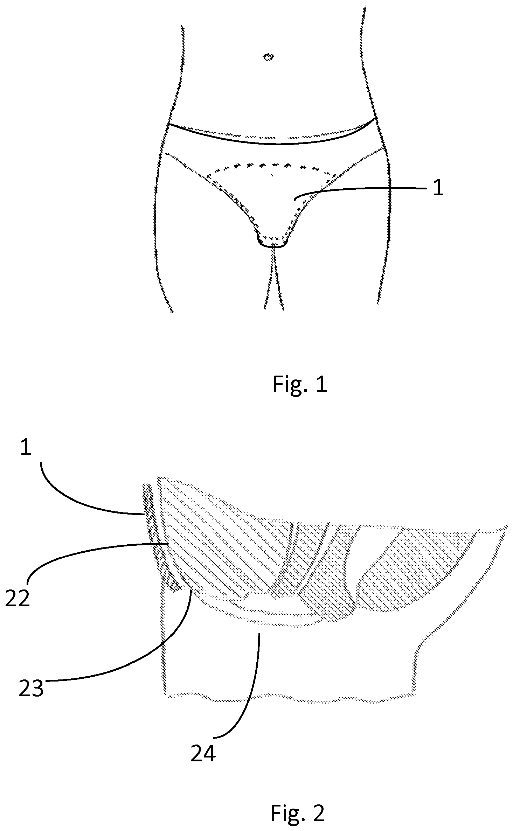

As indicated above, the veil is configured to be worn so that it covers the mons pubis in front of the anterior labia commissure of a female user, without covering any part of the vulval vestibule area of the user. The mons pubis, also known as mons Venus or mons veneris, is a rounded mass of fatty tissue found over the pubic symphysis of the pubic bones. In human females, the mons pubis forms the anterior portion of the vulva. It divides into the labia majora, on either side of the furrow known as the pudendal cleft that surrounds the labia minora, clitoris, urethra, vaginal opening, and other structures of the vulval vestibule. Figs. 1 and 2 show how the veil is to be worn. Fig. 1 illustrates how the veil is placed in a panty, such that it covers the mons pubis in front of the anterior labia commissure. Fig. 2 shows a cross section of the female anatomy of a wearer and illustrates how the veil 1 covers the mons pubis 22 in front of the anterior labia commissure 23, and how it does not cover any part of the vulval vestibule area 24.

The intimate skin conditioner veil is configured to have a shape that essentially follows the anatomy of the wearer in the genital area. This is accomplished by a main portion of the veil. The main portion preferably has the general shape of a truncated triangle, so that the veil is wider at a front end towards the abdomen of the user, and narrower at a rear end towards the vulval vestibule area. Although a truncated triangular main portion is preferred, the veil may alternatively have a non-truncated triangular shape, with the base of the triangle at the front end and the top of the triangle at the rear end. Further, the intimate skin conditioner veil is preferably very pliable and very thin, as will be described in further detail below.

The intimate skin conditioner veil comprises an additive composition in the form of a skin well-being agent or a carrier substance carrying a skin well-being agent. The term "skin well-being agent" is used herein to refer is a substance which has skin conditioning properties, and contributes to increase the comfort of the wearer. The additive composition thus preferably comprises one or more skin softening agents, or cooling agents, hydrating agents or combinations thereof.

The term "skin softening agent" is used therein to refer to a substance which is able to increase the moisture content in the skin when topically applied to the skin of the wearer, for example by depositing fat on the skin surface or occluding or adding moisture binding substances. An example of a suitable skin softening agent is occluding fats, such as, but not limited to, petrolatum (e.g. VASELINE®). The term "moisture binding substance" is used herein to refer to a substance which is able to bind moisture. Examples of suitable moisture binding substances that can be used in the additive composition are pidolic acid, urea, lactic acid, glycerol or propylene glycol, glycerine pantenol, allantoin or combinations thereof.

The skin well-being agent can consist of, but is not limited to, in its physical form, a solution, suspension, cream, lotion, ointment, paste, gel, foam, aerosol or capsule, or it can be present in solid phase as particles, flakes, fibres, films, foams, etc. In the following description, the terms cream, lotion or solution are used, but the other above-described forms are of course also conceivable.

Skin well-being agents can include lipids (including but not limited to fats, oils, waxes), solvents (including but not limited to water), water-soluble substances, surface-active agents (including but not limited to emulsifiers, surfactants), viscosity-regulating substances, pH-regulating substances, preserving agents, complexing agents (e. g. chelate), delivery systems (e. g. liposomes, microcapsules, etc.), pigments, perfumes, and active substances (including pharmaceutical agents). The lipids are usually emulsified in water, known as oil/water emulsion, or water is emulsified in the lipid phase, known as water/oil emulsion.

Lipid skin well-being agents can be paraffins (alkanes) with 12-35 carbons, such as, but not limited to, paraffin oil (mineral oil) or petrolatum (e.g., VASELINE®).

Other examples of suitable lipid skin well-being agents are triglycerides, which may be refined and/or hydrogenated, of animal or vegetable origin, preferably with carbon chain lengths C-18 or less, such as, but not limited to milk fat, coconut oil (Cocous nocifera), palm-kernel oil (Elaeis guineeis), or caprylic triglycerid; animal or vegetable lipids with unsaturated C-18 fatty acids, such as, but not limited to, Japan wax (Rhus succesdanes), tallow fat, soybean oil (Glycerin soya), peanut oil (Arachais hypogaea), maize oil (Zea mays), sunflower oil (Helanthus annus), grapeseed oil (Vitis vinifera), safflower oil (Carthamus tinctorius), sweet almond oil (Prunnus amygdalus dulcis), hazelnut oil (Corylus americana), walnut oil (Juglans regia), olive oil (Olea europasa), avocado oil (Persea gratissima), sesame oil (Sesamum indicum), cottonseed oil (Gopssypium), palm oil (Elaesis guineensis), rice oil (Oryza sativa), rape oil (Canola), apricot-kernel oil (Prunus armeniaca), cocoa butter (Theobroma cao), shea butter (Butyrospermum parkii), wheatseed oil (Triticum vulgare), or Bassia latifola oil; or animal or vegetable lipids with carbon chains over C-18, such as, but not limited to, beeswax Cera alba), seed oil (Limnanthes alba), rapeseed oil (Brassica capmestris), cucumberseed oil (Borago officinalis), linseed oil (Linum usitatissimum), ricin oil (Ricinus communis), veronia oil (Veronia galamensis), jojoba oil (Buxus chinensis), candlewax (Euphorbia cera), or ongokea oil (Ongokea gore).

The skin well-being agents may also be fatty alcohols with straight or branched carbon chain lengths of 12-32 carbons, for example, cetyl alcohol, stearyl alcohol or a mixture thereof, or fatty acid esters with 12-32 carbons, for example, methyl palmitate, methyl stearate, isopropyl myristate, isopropyl laurate, isopropyl palmitate, isopropyl stearate, octyl palmitate, octyl stearate or octyl laurate.

The skin well-being agents may also be polyalcohols, for example sugar alcohols or polyglycerols.

The skin well-being agents may also be complex lipids, for example, phospholipids or sphingolipids (ceramides); or waxes, for example of animal origin, for example beeswax or lanolin, or of vegetable origin, for example carnauba or candelilla, or of mineral origin, for example ozocerite or ceresin.

The skin well-being agents may also be polysiloxanes, which may be straight, branched or cyclic. Examples are polydimethyl-siloxane (dimethicone) or polydiethylsiloxane.

Skin well-being agents can include emulsions, such as, but not limited to, emulsions of one or more fat, with hydrophilic substances, such as, but not limited to, water, glycerol, polyethylene glycol (PEG), propylene glycol, butylene glycol, sorbitol, silicone glycols, or the like, or mixtures thereof.

The skin well-being agents can include pH-regulating additives, for example organic or inorganic acids, such as, but not limited to, adipic acid, ascorbic acid, benzoic acid, citric acid, malic acid, tartaric acid, lactic acid, phosphoric acid, or hydrochloric acid; or buffers, made for example from said acids with their corresponding salts. The skin well-being agents can also include polymeric acids, for example polyphosphoric acid or polyacrylic acid.

Skin well-being agents can also include additions of probiotic microorganisms, characterized by being antagonistic towards undesired microorganisms, e. g. skin infection pathogens. Examples of probiotic microorganisms which can be used are individual strains or mixtures of several strains of lactic acid bacteria taken from the species Lactobacillus acidophilus, Lactobacillus curvatus, Lactobacillus plantarum or Lactococis lactis.

Skin well-being agents can also include active substances, such as, but not limited to, anti-inflammatory agents, e. g. acetylsalicylic acid, allantoin, azulen, alpha- bisabolol (chamomile), flavonoids, glycyrrhizinic acid, ichthammol (Inotyol)), tannins, or astringents (vasoconstrictors), for example TiO, ZnO (and other Zn compounds), aluminum acetate solution, aluminum tartrate solution (and other Al compounds), ethanol or ethanol-based solutions.

Skin well-being agent can also include nourishing agents such as, but not limited to, Aloe vera (Aloe barbadensis), alpha-hydroxy acids, for example citric acid, tartaric acid, lactic acid, malic acid, etc.; or algae extract, ascorbic acid (vitamin C), or vitamin A compounds, for example retinol, retinal, tretinoin and isotretinoin, or avocado sterols, betaine (trimethylglycine), ceramides, grapeseed extract, essential fatty acids, flavonoids, phytosphingosine, phytosterols, hyaluronic acid, yeast extract, chitosan, milk protein (Lactis proteinum), pantenol (provitamin B5), polysaccharides, rosemary extract, tocopherol (vitamin E), ubiquinone (coenzyme Q10), urea.

Skin well-being agents can also include calendula officinalis flower extract, mannitol, ammonium glycyrrhizate, caffeine, zinc gluconate, aesculus hippocastanum extract, tocopheryl acetate.

Skin well-being agents can also consist of ready-made mixtures of skin ointments, creams and lotions, for example, TENA Skin Cream, which include the ingredients: Aqua, Canola Oil, Glycerin, Ethylhexyl Stearate, Glyceryl Stearates, Zea Mays Oil,Polyglyceryl-3 Methylglucose Distearate, Cetearyl Alcohol, Dicaprylyl Carbonate, Hydrogenated Coco-Glycerides, Panthenol, Tocopheryl Acetate, Hydroxyethyl Acrylate/Sodium Acryloyldimethyl Taurate Copolymer, Betaine, Parfum, Xanthan Gum, Sodium Citrate, Phenoxyethanol, Benzoic Acid, Dehydroacetic Acid, Tocopherol, Citric Acid, Sorbitan Isostearate, Polysorbate 60, Pantolactone; or TENA Skin Cream Perfume free which include the ingredients; Aqua, Canola Oil, Glycerin, Ethylhexyl Stearate, Glyceryl Stearates, Zea Mays Oil,Polyglyceryl-3 Methylglucose Distearate, Cetearyl Alcohol, Dicaprylyl Carbonate, Hydrogenated Coco-Glycerides, Panthenol, Tocopheryl Acetate, Hydroxyethyl Acrylate/Sodium Acryloyldimethyl Taurate Copolymer, Betaine, Xanthan Gum, Sodium Citrate, Phenoxyethanol, Benzoic Acid, Dehydroacetic Acid, Tocopherol, Citric Acid, Sorbitan Isostearate, Polysorbate 60, Pantolactone; or TENA Skin Lotion, which include the ingredients: Aqua, Dicaprylyl Carbonate, Polyglyceryl-3 Methylglucose Distearate, Ethylhexyl Stearate, Glycerin, Glyceryl Stearates, Canola Oil, Cetearyl Alcohol, Tocopheryl Acetate, Hydroxyethyl Acrylate/Sodium Acryloyldimethyl Taurate Copolymer, Panthenol, Parfum, Xanthan Gum, Sodium Citrate, Phenoxyethanol, Benzoic Acid, Dehydroacetic Acid, Tetrasodium Iminodisuccinate, Tocopherol, Citric Acid, Sorbitan Isostearate, Polysorbate 60, Pantolactone. TENA products are sold commercially by SCA Hygiene Products, Gothenburg, Sweden.

It should be noted that this disclosure is not limited to the skincare agents mentioned above, and that instead these are just examples of skincare agents that could be used.

The term "cooling agent" is used herein to refer to a substance which is able to convey a freshness or cooling sensation when topically applied to the skin of the wearer.

The cooling agents suitable for use herein include, but not limited to, all cooling agents being able to penetrate skin barrier and for which the cooling effect (also called herein freshness effect) is a physiological effect due to the direct action of these agents on the nerve endings of the body responsible for the detection of cold without any occurrence of temperature change on the surface of the body. It is believed that these agents act as a direct chemical stimulus on the cold receptors at the nerve endings, which in turn stimulate the central nervous system. In this way a freshness/cooling sensation is simulated even in absence of real change in skin temperature. Due to the persistence of the stimulus, a long lasting freshness/cooling sensation is delivered even after removal of the cooling agent. It is to be understood herein that the freshness/cooling sensation is personnel to a given individual. It must be admitted that skin tests are somewhat subjective, some individuals experiencing a greater or lesser freshness/cooling sensation than others when subjected to the same test. This perception depends on the density of thermo-receptors on skin and on the skin thickness. Typically it is observed that the thinner the skin is the more intense is the cooling sensation (also called herein freshness sensation). The thinner the skin is, the more rapid is the penetration of the cooling agent through the skin and higher is the absorption level thereof. Furthermore, studies have demonstrated that geographic factors and/or races further play a role in perception of freshness sensation.

Examples of suitable cooling substances are selected from the group consisting of ketals, carboxamides, cyclohexyl derivatives, cyclohexanol derivatives, menthol derivatives, camphor, borneol, eucalyptol, methyl salicylate, tea tree oil, eucalyptus oil, menthol or menthyl lactate or menthone glycerin aceta, bisabolol or mixtures thereof.

The term "carrier substance" is used herein to refer to a substance which is able to act as a carrier vehicle for delivering an effective concentration of a skin well-being agent to a wearer's skin. Suitable carrier substances include, but are not limited to, polyethylene glycol, propylene glycol, water, dimethcone, oil, petrolatum (e.g., VASELINE®) or alcohol, or combinations thereof.

PEGs are typically used in the composition as a carrier vehicle for delivering an effective concentration of a cooling agent to a wearer's skin but since PEGs are also emollients, they are particularly beneficial to skin, they improve skin hydration and softness, and hence maintain or even improve skin health. PEGs assure a film-forming capacity on the skin, which gives emolliency and helps prevent skin dehydration when directly contacting the skin, thereby reducing or even eliminating the occurrence of skin itching or burning. PEGs are also able to locate themselves between the layers of the epidermis (due to their similarity with substances naturally contained in the epidermis (stratum corneum)), enhancing thereby the elastic properties of the skin.

PEGs having a weight average molecular weight of 2000 Da or more are not only acting as a carrier vehicle for delivering for example the cooling agent to the skin but also help to increase the stability of the cooling agent in the composition, i.e. to retain the cooling agent into the composition and therefore reduce the risk that the cooling agent may vaporize and evaporate at room temperature.

The additive composition used in the skin conditioner veil a sufficient amount of the cooling agent to stimulate the thermo-receptors in the areas of the skin and/or mucosal surfaces with which the article comes into contact and thereby convey the desired freshness sensation.

The additive composition may also include, but is not limited to, solvents, emulsifying agents, fragrances, and preservatives.

The additive composition comprising the skin well-being agent is preferably located on the skin contacting side of the veil, and is suitably comprised within the space creating layer, for example in a loft layer or a loft portion of the space creating layer. The additive composition can be applied to the veil by impregnating the loft layer and/or the top sheet with the composition, or it can be applied on the skin contacting surface of the top sheet, for example by spraying, slot coating or printing. The additive composition may also be applied evenly over the surface area of the veil, or may be applied in a pattern, such as a pattern dots, stripes etc. on the skin contacting surface/top sheet of the veil.

The additive composition comprising the skin well-being agent is advantageously present in the intimate skin conditioner veil in a combined amount of skin well-being agent and any carrier substance of 1-300 wt-%, preferably 1-150 wt-%, more preferably 1-50 wt-%, based on the total weight of the intimate skin conditioner veil.

When the skin well-being agent is carried by polyethylene glycol as carrier substance, the combined amount of skin well-being agent and polyethylene glycol is preferably 1-100 wt-%, based on the total weight of the intimate skin conditioner veil, more preferably 5-30 wt-%.

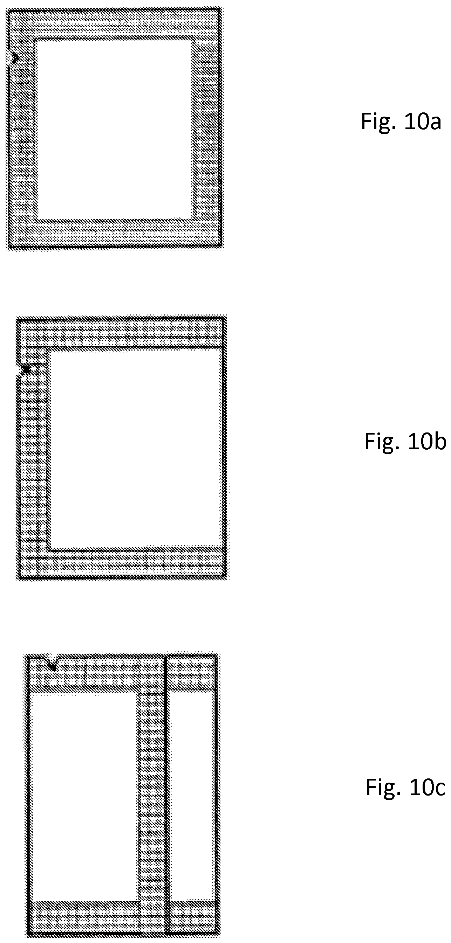

The veil may be in the form of a packaged intimate skin conditioner veil, wherein the veil is enclosed in a wrapping material, which is impermeable to the skin well-being agent. Figs. 10a-c show packages, in which a wrapping material is wrapped around the intimate skin conditioner veil and sealed along one or more edges, by welding or adhesive, or any other suitable means. The intimate skin conditioner veil can be in flat unfolded condition in the package, or it can be folded once.

The veil can be stacked and wrapped in package material, or be individually packed. The wrapping material is preferably air-tight, and thus moisture and water tight. The material is preferably in the form of a film comprising one or more polymers, such as, but not limited to, polyethylene, polypropylene, polyester, polyamide, polyvinyl alcohol or similar polymers, or an aluminum foil, aluminum oxide or silicone oxide or the like, or combinations thereof. The water vapour transmission rate of such a material is 0-6 g/m²/calendar day according to ASTME 398-83 at 37.8 °C and 90% relative humidity, preferably 0-2 g/m²/calendar day and even more preferably 0-1 g/m²/calendar day. The films may have a thickness of 10-200 micrometers, preferably 20-100 micrometers.

The film wrapping material can also be laminated with other layers such as, but not limited to, nonwoven material or sheet of tissue. The wrapping material can be cold or hot sealed, with or without use of adhesives.

### Outer contour

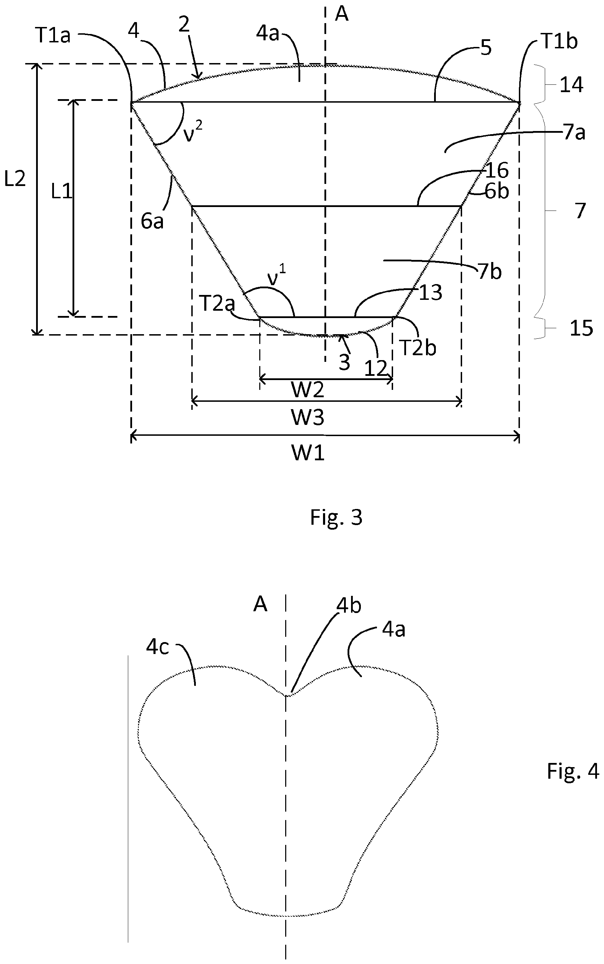

Fig. 3 illustrates an example of an intimate skin conditioner veil of the present disclosure. The veil has a longitudinal central line A extending in the longitudinal direction of the veil; a first extension in the longitudinal direction between a front end 2 and a rear end 3 of the veil; and a second extension in a transverse direction perpendicular to the longitudinal central line A. It comprises a front edge 4 and first and second side edges 6a, 6b, the front edge 4 intersecting the first side edge 6a at a first front transition point T1a and intersecting the second side edge 6b at a second front transition point T1b. The main portion 7 of the veil has a length L1 in the longitudinal direction of the veil, which is at least 50% of the total longitudinal length L2 of the veil 1. The main portion 7 is bounded by a straight front transversal line 5 between the first and second front transition points T1a, T1b, and by the first and second side edges 6a, 6b. The main portion 7tapers towards the rear end 3 of the veil. The main portion 7 is reflection symmetric about the longitudinal central line A. As indicated above, the main portion 7 is wider at its front end than at its rear end, and it preferably has a front transversal width W1 and a rear transversal width W2 chosen such that the ratio W1/W2 is 1.1-6.6, preferably 1.5-4.2, and more preferably 2.8-3.5.

The intimate skin conditioner veil 1 may further comprise a rear edge 12 which extends substantially in the transverse direction of the veil 1, at the rear end 3 of the veil, the rear edge 12 intersecting the first side edge 6a at a first rear transition point T2a and intersecting the second side edge 6b at a second rear transition point T2b. The first and second side edges 6a, 6b extend between respective ones of the first and second front transition points T1a, T1b and respective ones of the rear transition points T2a, T2b, and the main portion 7 extends in the longitudinal direction between the front transversal line 5 and a straight rear transversal line 13 between the first and second rear transition points T2a, T2b, and the straight rear transversal line 13 is perpendicular to the longitudinal central line A.

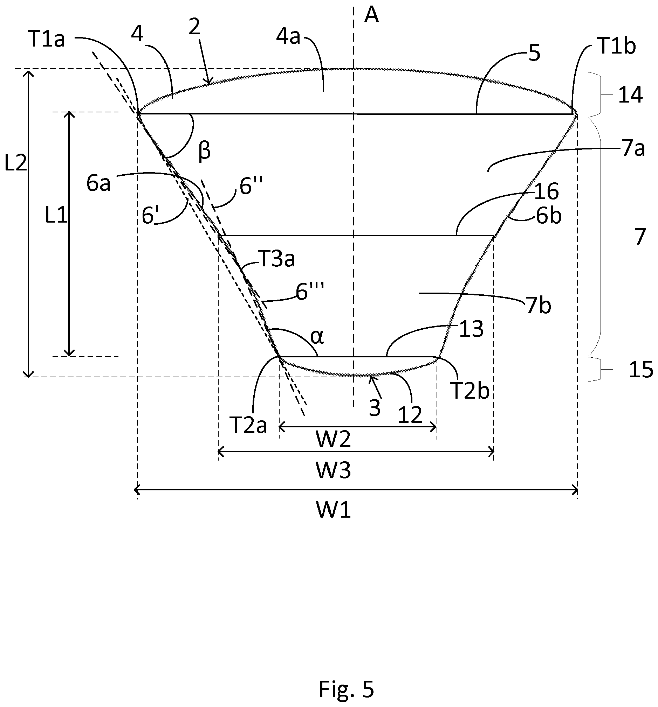

The first and second front transition points T1a, T1b and the first and second rear transition points T2a, T2b are located where the outer contour of the veil dramatically changes direction, between a generally longitudinal direction and a generally transversal direction. The front edge 4 of the veil meets the side edges 6a, 6b at the first and second front transition points T1a, T1b, respectively, and the rear edge 12 meets the side edges 6a, 6b at the first and second rear transition points T2a, T2b, respectively, such that the transition points form corners between the front and rear edges 4, 12 and the side edges 6a, 6b. These corners at the transition points can have a sharp shape or may be rounded. The veil shown in Fig. 3 has sharp corners at the front transition points, and the veil shown in Fig. 5 has rounded corners at the front transition points.

The intimate skin conditioner veil 1 may include a head portion 14 at the front end of the veil 1 and/or a tail portion 15. The head portion 14 extends between the front straight line 5 and the front edge 4 of the veil 1. The front edge 4 of the veil 1 is then also the front edge of the head portion 14. The head portion 14 can have various shapes, and can comprise at least one protuberance 4a, which may have a convex shape, as shown in Fig. 3. The head portion 14 may also comprise at least one recess 4b, which may have a concave shape, and which may be positioned symmetrically about the longitudinal central line A, as shown in Fig. 4. The head portion 14 may include at least one protuberance 4a, 4c located on each side of the recess 4b. Fig. 4 illustrates an example of a veil, in which one protuberance 4a, 4c is located on each side of a centrally positioned recess 4b, giving the veil 1 an outer contour resembling a rounded heart. Alternatively, a larger number of protuberances and recesses can be comprised in the head portion 14, for example, but not limited to 1-10 protuberances, and 1-9 recesses. The tail portion 15 extends between the rear transversal line 13 and the rear edge 12. The tail portion 15 portion can have various shapes, and may for example comprise at least one protuberance 12a and/or recess. The head and tail portions 14, 15 serve to further adapt the veil 1 to the anatomy of the wearer, by providing curved front and rear end contours. Fewer protuberances and recesses are preferred for manufacturing reasons, since a veil having a low number of protuberances and recesses in head or veil portion, may lead to a less complicated manufacturing process, while more protuberances and recesses provide a softer edge of the veil, such that the contour of the veil can follow the body shape of the user in an improved manner.

When the veil 1 includes a head portion 14 and/or a tail portion 15, the total longitudinal length L2 of the veil is the combined longitudinal length L1 of the main portion and the longitudinal lengths of the head portion and/or the tail portion. The ratio L1/L2 is preferably 0.5-1, more preferably 0.6-0.9, most preferably 0.75-0.85 so as to follow the anatomy of the wearer. The longitudinal length L1 of the main portion 7 is preferably 60-170 mm, and the total longitudinal length L2 of the veil is 60-220 mm, more preferably 80-150 mm, most preferably 90-120 mm so as to follow the anatomy of the wearer. The main portion preferably has a front transversal width W1 of 100-240 mm, more preferably 140-210 mm, most preferably 155-175 mm and a rear transversal width W2 of 25-95 mm, more preferably 40- 80 mm, most preferably 55-70 mm so as to follow the anatomy of the wearer. By choosing the length and width of the veil according to the anatomy of the wearer, it can be ensured that the main portion is sufficiently large to cover the mons pubis in front of the anterior labia commissure.

In order to follow the anatomy of the wearer more closely, the main portion 7 may be tapered such that a straight line 6' between the first front transition point T1a and the first rear transition point T2a is inclined in relation to the front transversal line 5 at an angle v², which is smaller than 90°, preferably 20-85°, more preferably 50-80°, most preferably 55-75°. The line 6' is inclined in relation to the rear transversal line 13 at an angle v¹, which is greater than 90°, preferably 95-160°, more preferably 100-130°, most preferably 105-125°, such that v² =180° - v¹.

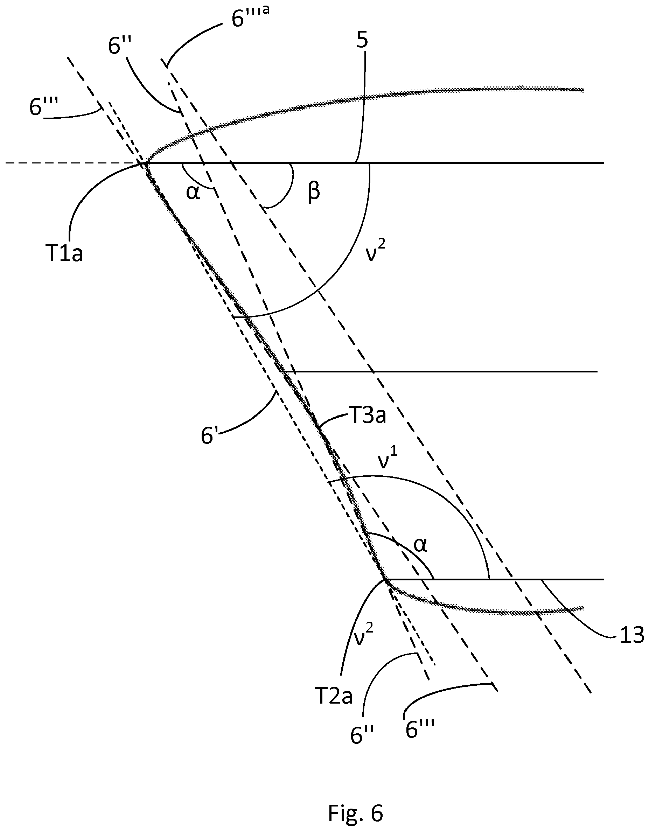

In order to further improve the fit of the veil in accordance to the wearer's anatomy, the first and second side edges 6a, 6b of the veil may advantageously have a curved concave shape between the front and rear transition points T1a, T1b, T2a, T2b. The examples of veils shown in Figs. 4-6 have such curved side edges. A preferred curvature is shown in more detail in Figs. 5 and 6.

As illustrated in Fig. 5, the main portion 7 of the intimate skin conditioner veil preferably comprises a front main portion 7a located between the front transversal line 5 and a straight intermediate transversal line 16, and a rear main portion 7b located between the rear transversal line 13 and the intermediate transversal line 16. The intermediate transversal line 16 is located at the longitudinal centre of the main portion, such that the longitudinal length of the front main portion 7a is the same as the longitudinal length of the rear main portion 7b. The curved concave shapes of the first and second side edges 6a, 6b preferably change curvature at respective intermediate transition points to adopt respective directions of curvature and continue to run in the same respective directions of curvature. For example, Fig. 5 shows that the curved concave shape of the first side edge 6a changes curvature at an intermediate transition point T3a, and continues to run in the same direction of curvature, and the intermediate transition point T3a is located in the rear main portion 7b between the intermediate transversal line 16 and the rear transversal line 13. The second side edge 6b has a similar shape. The main portion preferably has an intermediate transversal width W3 of 65-90 mm at the intermediate transversal line 16, in order to give a good fit to the wearer's anatomy.

The fit to the anatomy of the wearer can be further configured to adapt the curvature of the side edges of the veil to the groin anatomy of the wearer. As illustrated in Figs. 5 and 6, the first and second side edges 6a, 6b may thus advantageously have a curvature, which changes such that a straight line 6" between the first rear transition point T2a and the intermediate transition point T3a, is inclined in relation to the rear transversal line 13 at an angle α, which is > 90°, and a straight line 6"' between the first front transition point T1a and the intermediate transition point T3a is inclined in relation to the front transversal line 5 at angle β, which is < 180° -α. Further, the angle α is preferably smaller than the angle v¹ between the rear transversal line 13 and the straight line 6'between the first rear transition point T2a and the first front transition point (T1a), and the angle β is preferably smaller than the angle v² between the straight line 6' and the front transversal line 5. The angle α is preferably 95-160°. A line 6"'^{a}, which is parallel to the line 6"', is drawn in Fig. 6 for illustration purposes.

### Materials

In order to conform well to the body of the wearer and to add to the wearing comfort, the intimate skin conditioner veil 1 is preferably very pliable, thin and breathable. The veil comprises a garment facing layer arranged on the garment facing side of the veil, and a space creating layer arranged on the skin contacting side of the veil. The layers comprised in the veil are suitably connected by adhesive or ultrasound welded joints.

The intimate skin conditioner veil may include fastening means positioned on the garment facing side, preferably in the form of one or more adhesive or friction areas.

The pliability, stiffness of the intimate skin conditioner veil 1 can be expressed as Gurley Units, and the veil 1 preferably has a Gurley Units of 5-300 mgf, more preferably 5-100 mgf. Gurley Units are measured according to the standard method EDANA/INDA NWSP 090.2.RO (15) modified for measuring the stiffness of an intimate skin conditioner veil product, as described in more detail below. The desired pliability can preferably be obtained by choosing the layers of the veil as described below.

The intimate skin conditioner veil 1 preferably has a total thickness of 0.6-3.8 mm. The space creating layer of the veil may be a combined layer, comprising a loft layer and a top sheet. The loft layer of the space creating layer preferably has a basis weight of 15-100 gsm and a thickness of 0.5-3.5 mm.

The top sheet is preferably a nonwoven material comprising natural and/or synthetic fibres, and preferably has a basis weight of 8-100 gsm, which may be perforated or embossed. Other examples of suitable top sheet materials include, but are not limited to, perforated plastic films, plastic or textile mesh, and fluid permeable layers of foam, such as polyurethane foam based on polyester. Laminates consisting of two or more top sheet material may also be employed, and the top sheet material may be different in different parts of the skin-contacting surface.

The loft layer preferably comprises hydrophobic or hydrophilic nonwoven material, apertured thermoplastic film or open foam material. The nonwoven material may be for example, but is not limited to, air through bonded nonwoven, spunbond, SMS material (spunbond/meltblown/spunbond), carded thermobonded nowoven, or spunlaced (hydroentangled) material.

Alternatively, the space creating layer may be a multilayer layer comprising a loft portion and a top sheet portion, the loft portion being positioned between the garment facing layer and the top sheet portion. The space creating layer may then be a nonwoven material comprising at least two layers integrated into each other, wherein the loft portion preferably comprises spunlaid filaments having a thickness greater than 2.5 dtex, giving an open structure to the loft portion, and the top sheet portion preferably comprises staple fibres, giving a soft feeling. A suitable multilayer layer is described in WO2008147264A1. Such a multilayer space creating layer preferably has a basis weight of 15-100 gsm and a thickness of 0.5-3.5 mm.

The loft layer or loft portion of the space creating layer provides an open structure, which contributes to forming a distance between the skin and the garments of the wearer. The loft layer or loft portion can thus function as a replacement for the hair that has been removed, and can thus contribute to airiness and favourable skin conditions in the genital area, particularly in the V-zone. All layers included in the veil can have the same size, or the loft may be smaller than the garment facing layer and the top sheet. With a smaller loft layer, the veil comprises an edge portion along the outer contour, comprising only the garment facing layer and the top sheet, thus making the edges of the veil even thinner and softer.

The garment facing layer is suitably breathable, and preferably comprises a breathable plastic film or a nonwoven material, or a laminate including a breathable plastic film and a layer of nonwoven material. The garment facing layer is preferably also liquid-tight, which means that the material can resist the flow of liquid. Suitable materials for the garment facing layer include, but are not limited to, perforated films, microporous films, macroporous films, nanoporous films, or nowoven or laminates thereof. Examples of nonwoven laminates include, but are not limited to, laminates of spunbond and meltblown, for example SMMS laminate (spunbond/meltblown/spunbond-laminate). The garment facing layer material can include, but is not limited to, renewable material in the form of PLA starch or the like.

The intimate skin conditioner veil 1 preferably does not affect the humidity conditions that prevail in the intermediate space between the skin and the veil more than normal cotton briefs. Each layer of the intimate skin conditioner veil 1 advantageously has a moisture vapour transmission rate greater than 1000 g/m²/24h, and the intimate skin conditioner veil preferably has a total moisture vapour transmission rate greater than 1000 g/m²/24h, preferably 1000-6000 g/m²/24h, more preferably 1300-1600 g/m²/24h. The garment facing layer thereby allows perspiration generated by the skin to be able to leave the surface of the skin, which reduces moisture on the skin surface and thereby reduces the risk for undesirable skin conditions. The garment facing layer suitably has a basis weight of 18-21 gsm.

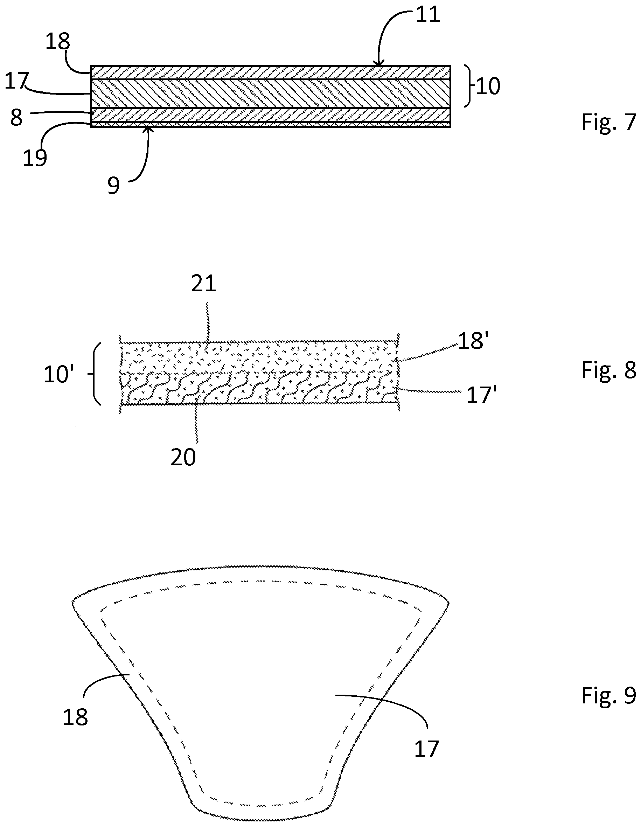

Fig. 7 shows a schematic cross sectional view of the veil. The veil 1 has a garment facing side 9 and a skin contacting side 11, the garment facing side 9 and the skin contacting side 11 facing away from one another. The veil 1 comprises a garment facing layer in the form of a back sheet 8 arranged on the garment facing side 9 of the veil, and a space creating layer 10, arranged on the skin contacting side 11 of the veil. Fastening means 19 are arranged on the garment facing side 9 of the veil. In the example shown in Fig. 7, the space creating layer is a combined layer 10, comprising a loft layer 17 and a top sheet 18, and the loft layer 17 is positioned between the garment facing layer 8 and the top sheet 18. Fig. 8 illustrates a multilayer space creating layer 10' that can be used instead of the combined space creating layer 10 shown in Fig. 7. The multilayer space creating layer 10' comprises a loft portion 17' comprising spunlaid filaments 20 and a top sheet portion 18' comprising staple fibres 21. The multilayer space creating layer 10' is arranged in the veil such that the loft portion 17' is positioned between the garment facing layer 8 and the top sheet portion 18'. Fig. 9 is a schematic top view, showing an example of an intimate skin conditioner veil in which the loft layer 17 is smaller than the top sheet 18 and the back sheet (not shown in Fig. 9).

### Measuring methods

### Stiffness

All values for thickness given above for the layers comprised in the intimate skin conditioner veil are obtained by measuring by means of a circular measuring foot having a diameter of 31.5 mm at pressure of 0.2 kPa.

Gurley stiffness is measured according to the standard method EDANA/INDA NWSP 090.2.RO (15). This method is originally intended for measuring stiffness of nonwoven materials, and is therefore modified for measuring stiffness of the entire intimate skin conditioner veil. The method accordingly includes the following additional steps:
- remove any release paper from the veil, and cover the attachment adhesive lightly with talcum, or the other equivalent powder;
- cut a representative sample from the center region of the veil, avoiding any folds; and
- if the stiffness of the veil is different along the longitudinal and the transverse axel, let the lowest value represent the article.

### Moisture Vapour Transmission Rate

The Moisture Vapour Transmission Rate is a measure of the amount of moisture adsorbed by Calcium-Chloride in a "cup" like container covered with the test specimen from controlled outside air conditions (40±3 °C/ 75±3 % relative humidity). The sample holding a cup is a cylinder with an inner diameter of 30 mm and an inside height from bottom to top flange of 49 mm. A flange having a circular opening to match the opening of the cylinder can be fixed by screws, and a silicone rubber sealing ring, matching the inner diameter, fits between the top flange and the cylinder. The test specimen is to be positioned such that it covers the cylinder opening, and can be tightly fixed between the silicone rubber sealing and the upper flange of the cylinder. The test specimen is positioned on the cylinder with the garment facing layer facing the Calcium-Chloride. When testing an intimate skin conditioner veil having adhesive areas applied to the garment facing layer, a plurality (at least 5) test specimens are cut from the veil and tested, and a value representing the MVTR for the entire product is obtained by calculating an average MVTR value of the individual test specimens. The equipment as well as the test specimen should be well adjusted to the temperatures, and the constant temperature/humidity chamber preferably has a size to accommodate up to 30 samples. The absorbent desiccant material is CaCl₂, such as can be purchased from Wako Pure Chemical Industries Ltd., Richmond, VA, US under the product designation 030-00525. If kept in a sealed bottle, it can be used directly. It also can be sieved to remove lumps, or excessive amounts of fines, if existing. It also can be dried at 200 °C for about 4 hrs. 15.0± 0.02 g of CaCl₂ are weighed into the cup, and tapped lightly so as to level it out, such that the surface is about 1 cm from the top of the cup.

The samples, which are cut to about 3.2 cm by 6.25 cm, are placed flat and overlapping with the seal over the opening, and the seal and the top flange are affixed by the screws without over tightening. The total weight of the cup assembly is accurately recorded on a four decimal places scale, and the assembly is placed into the constant temperature/humidity chamber. After 5 hrs (without opening of the chamber), the sample is removed and immediately covered tightly with non-vapour permeable plastic film such as SARAN wrap as commonly used in the U.S. After about 30 mins to allow for temperature equilibration, the plastic film cover is removed and the accurate weight of the assembly is recorded. The MVTR value is then calculated from the moisture increase during these 5 hours through the 3 cm circular opening and then converted to units of g/m²/24h. For each test, three replicates should be run, the resulting values will be averaged, and the result rounded to the nearest 100 value.

Overall, this method is applicable to thin films, multi-layer laminates and the like. Experience has shown that typical standard deviations range between 50 and 250 g/m²/24h for averaged values of up to about 5000 g/m²/24h. Due to this range, materials being considered to be essentially vapour impermeable, such as conventional PE films, are reported as having a MVTR of about 200 g/m²/24h. If the units for an MVTR value are omitted for simplicity, a material "having a MVTR value of 1000" should accurately be a material "having a MVTR value of 1000 g/m²/24h" according to this method.

The presently disclosed embodiments are considered in all respects to be illustrative and not restricted. The scope of the invention is indicated by the appended claims rather than the foregoing description and all changes that come within the meaning and range and equivalence thereof are intended to be embraced therein.

## Claims

1. An intimate skin conditioner veil (1), configured to be positioned in an undergarment so that it covers a mons pubis in front of an anterior labia commissure of a female user, without covering any part of a vulval vestibule area of the user, wherein
the veil is substantially non-absorbent, being configured to absorb 0.9% by weight sodium chloride solution in an amount of 0-2 times its own weight, and
the veil (1) has a longitudinal central line (A) extending in the longitudinal direction of
the veil (1), and a first extension in the longitudinal direction, between a front end (2) and a rear end (3) of the veil, and a second extension in a transverse direction perpendicular to the longitudinal central line (A), and
wherein the veil (1) comprises a front edge (4) and first and second side edges (6a, 6b), the front edge (4) intersects the first side edge (6a) at a first front transition point (T1a) and intersects the second side edge (6b) at a second front transition point (T1b), the veil (1) further comprises a main portion (7) having a length (L1) in the longitudinal direction of the veil, the veil (1) has a total longitudinal length (L2), the length (L1) of the main portion (7) being at least 50% of the total longitudinal length (L2) of the veil (1), wherein the main portion (7) is bounded by a straight front transversal line (5) between the first and second front transition points (T1a, T1b) and by the first and second side edges (6a,6b), and tapers towards the rear end (3) of the veil, the main portion (7) being reflection symmetric about the longitudinal central line (A),
wherein the veil (1) has a garment facing side (9) and a skin contacting side (11), the garment facing side (9) and the skin contacting side (11) face away from one another, and the veil (1) comprises a garment facing layer (8) arranged on the garment facing side (9) of the veil (1), and a space creating layer (10, 10') arranged on the skin contacting side (11) of the veil (1), the space creating layer (10, 10') comprising a loft layer (17) and a top sheet (18), the loft layer (17) being positioned between the garment facing layer (8) and the top sheet (18), or a loft portion (17') and a top sheet portion (18'), the loft portion (17') being positioned between the garment facing layer (8) and the top sheet portion (18'), and
wherein the veil (1) further comprises an additive composition in the form of either: (i) a skin well-being agent; or (ii) the skin well-being agent and a carrier substance carrying the skin well-being agent, and
wherein the additive composition comprising the skin well-being agent is comprised in the loft layer (17) or the loft portion (17') of the space creating layer (10, 10').

2. The intimate skin conditioner veil (1) of claim 1, wherein the additive composition comprises at least one skin softening agent, and/or at least one hydrating agent, and/or at least one cooling agent, and/or at least one pH regulating agent, and/or at least one nourishing agent and/or at least one replenishing agent.

3. The intimate skin conditioner veil (1) of claim 2, wherein the additive composition comprises the at least one skin softening agent in the form of an occluding fat.

4. The intimate skin conditioner veil (1) of any one of claims 2-3, wherein the additive composition comprises the at least one skin softening agent in the form of moisture binding substances.

5. The intimate skin conditioner veil (1) of any of claims 2-4, wherein the additive composition comprises the at least one cooling agent.

6. The intimate skin conditioner veil (1) of any one of claims 1-4, wherein the carrier substance includes at least one of: propylene glycol, water, dimethicone, oil, petrolatum, and alcohol.

7. The intimate skin conditioner veil (1) of any one of claims 1-6, further comprising a rear edge (12) which extends substantially in the transverse direction of the veil (1), at the rear end (3) of the veil (1), the rear edge (12) intersecting the first side edge (6a) at a first rear transition point (T2a) and intersecting the second side edge (6b) at a second rear transition point (T2b), whereby the first and second side edges (6a, 6b) extend between respective ones of the front transition points (T1a, T1b) and respective ones of the rear transition points (T2a, T2b), and the main portion (7) extends in the longitudinal direction between the front transversal line (5) and a straight rear transversal line (13) between the first and second rear transition points (T2a, T2b), wherein the straight rear transversal line (13) is perpendicular to the longitudinal central line (A).

8. The intimate skin conditioner veil (1) of any one of claims 1-7, wherein the main portion has a front transversal width (W1) and a rear transversal width (W2), wherein the ratio W1/W2 is 1.1-6.6, preferably 1.5-4.2, more preferably 2.8-3.5, and wherein the front transversal width (W1) is 100-240 mm, preferably 140-210 mm, more preferably 155-175 mm, and the rear transversal width (W2) is 25-95 mm, more preferably 40- 80 mm, most preferably 55-70 mm.

9. The intimate skin conditioner veil (1) of any one of claims 1-8, wherein the ratio L1/L2 is 0.5-1, preferably 0.6-0.9, more preferably 0.75-0.85, and wherein the longitudinal length (L1) of the main portion (7) preferably is 60-170 mm, and the total longitudinal length (L2) of the veil preferably is 60-220 mm, preferably 80-150 mm, more preferably 90-120 mm.

10. The intimate skin conditioner veil (1) of any one of claims 1-9, wherein the main portion (7) is tapered such that a straight line (6') between the first front transition point (T1a) and the first rear transition point (T2a), is inclined in relation to the front transversal line (5) at an angle (v₂), which is smaller than 90°, preferably 20-82°, more preferably 50-80°, most preferably 55-75°.

11. The intimate skin conditioner veil (1) of any one of claims 1-10, wherein the first side edge (6a) has a curved concave shape between the first front transition point (T1a) and the first rear transition points (T2a), and the second side edge (6b) has a curved concave shape between the second front transition point (T1b) and the second rear transition points (T2b), and wherein the main portion (7) preferably comprises a front main portion (7a) located between the front transversal line (5) and a straight intermediate transversal line (16), and a rear main portion (7b) located between the rear transversal line (13) and the intermediate transversal line (16), and wherein a longitudinal length of the front main portion (7a) is the same as a longitudinal length of the rear main portion (7b), and wherein the curved concave shapes of the first and second side edges (6a, 6b) each change curvature at respective intermediate transition points (T3a) to adopt respective directions of curvature, and continue to run in the same respective directions of curvature, wherein the respective intermediate transition points (T3a) are located in the rear main portion (7b) between the intermediate transversal line (16) and the rear transversal line (13), and the main portion preferably has an intermediate transversal width (W3) of 65-90 mm at the intermediate transversal line (16).

12. The intimate skin conditioner veil (1) of claim 11, wherein the first and second side edges (6a, 6b) change curvature such that a straight line (6") between the first rear transition point (T2a) and the intermediate transition point (T3a), is inclined in relation to the rear transversal line (13) at an angle (α), which is > 90°, and a straight line (6''') between the first front transition point (T1a) and the intermediate transition point (T3a) is inclined in relation to the front transversal line (5) at angle (β), which is < 180° -(α).

13. The intimate skin conditioner veil (1) of claim 12, wherein the angle (α) is smaller than the angle (v₁) between the rear transversal line (13) and the straight line (6') between the first rear transition point (T2a) and the first front transition point (T1a), and the angle (β) is smaller than the angle (v₂) between the straight line (6') and the front transversal line (5).

14. The intimate skin conditioner veil (1) of any one of claims 1-13, having a Gurley stiffness of 5-300 mgf, more preferably 5-100 mgf.

15. The intimate skin conditioner veil (1) of any one of claims 1-14, wherein the space creating layer is a combined layer (10) comprising the loft layer (17) and the top sheet (18), and wherein the loft layer (17) of the space creating layer (10) preferably has a basis weight of 15-100 gsm and a thickness of 0.5-3.5 mm.

16. The intimate skin conditioner veil (1) of any one of claims 1-14, wherein the space creating layer is a multilayer layer (10') comprising the loft portion (17') and the top sheet portion (18').

17. The intimate skin conditioner veil (1) of claim 3, wherein the occluding fat includes petrolatum.

18. The intimate skin conditioner veil (1) of claim 4, wherein the moisture binding substances include pidolic acid, urea, lactic acid, glycerol, or propylene glycol.

19. The intimate skin conditioner veil (1) of claim 5, wherein the cooling agent includes ketals, carboxamides, cyclohexyl derivatives, cyclohexanol derivatives, menthol derivatives, camphor, borneol, eucalyptol, methyl salicylate, tea tree oil, or eucalyptus oil.

## Patentansprüche

1. Intimhautpflegeschleier (1), der dazu gestaltet ist, in einer Unterwäsche positioniert zu werden, sodass er einen Schamhügel vor einer Verbindungsstelle der großen Schamlippen einer weiblichen Nutzerin bedeckt, ohne irgendeinen Teil eines Scheidenvorhofbereichs der Nutzerin zu bedecken, wobei
der Schleier im Wesentlichen nicht absorbierend ist, wobei er dazu gestaltet ist, eine 0,9-gewichtsprozentige Natriumchloridlösung in einer Menge von 0 bis 2-mal seines eigenen Gewichts zu absorbieren, und
der Schleier (1) eine Längsmittellinie (A), die sich in der Längsrichtung des Schleiers (1) erstreckt, und eine erste Erstreckung in der Längsrichtung zwischen einem vorderen Ende (2) und einem hinteren Ende (3) des Schleiers und eine zweite Erstreckung in einer zu der Längsmittellinie (A) senkrechten Querrichtung hat, und
wobei der Schleier (1) eine Vorderkante (4) und erste und zweite Seitenkanten (6a, 6b) aufweist, die Vorderkante (4) die erste Seitenkante (6a) an einem ersten Vorderübergangspunkt (T1a) schneidet und die zweite Seitenkante (6b) an einem zweiten Vorderübergangspunkt (T1b) schneidet, der Schleier (1) ferner einen Hauptabschnitt (7) aufweist, der eine Länge (L1) in der Längsrichtung des Schleiers hat, der Schleier (1) eine Gesamtlängslänge (L2) hat, wobei die Länge (L1) des Hauptabschnitts (7) mindestens 50% der Gesamtlängslänge (L2) des Schleiers (1) ist, wobei der Hauptabschnitt (7) durch eine gerade Vorderquerlinie (5) zwischen den ersten und zweiten Vorderübergangspunkten (T1a, T1b) und durch die ersten und zweiten Seitenkanten (6a, 6b) begrenzt ist und sich in Richtung des hinteren Endes (3) des Schleiers verjüngt, wobei der Hauptabschnitt (7) um die Längsmittellinie (A) spiegelsymmetrisch ist,
wobei der Schleier (1) eine wäschezugewandte Seite (9) und eine hautberührende Seite (11) hat, die wäschezugewandte Seite (9) und die hautberührende Seite (11) voneinander abgewandt sind und der Schleier (1) eine wäschezugewandte Schicht (8), die an der wäschezugewandten Seite (9) des Schleiers (1) angeordnet ist, und eine raumerzeugende Schicht (10, 10') aufweist, die an der hautberührenden Seite (11) des Schleiers (1) angeordnet ist, wobei die raumerzeugende Schicht (10, 10') eine Speicherschicht (17) und eine Oberdecke (18), wobei die Speicherschicht (17) zwischen der wäschezugewandten Schicht (8) und der Oberdecke (18) liegt, oder einen Speicherabschnitt (17') und einen Oberdeckenabschnitt (18') aufweist, wobei der Speicherabschnitt (17') zwischen der wäschezugewandten Schicht (8) und dem Oberdeckenabschnitt (18') liegt, und
wobei der Schleier (1) ferner ein Zusatzgemisch aufweist, in der Form entweder: (i) eines Hautwohlwirkstoffes; oder (ii) des Hautwohlwirkstoffes und einer Trägersubstanz, die den Hautwohlwirkstoff trägt, und
wobei das Zusatzgemisch, das den Hautwohlwirkstoff aufweist, in der Speicherschicht (17) oder dem Speicherabschnitt (17') der raumerzeugenden Schicht (10, 10') enthalten ist.

2. Intimhautpflegeschleier (1) gemäß Anspruch 1, wobei das Zusatzgemisch mindestens einen Hautweichmachwirkstoff und/oder mindestens einen hydrierenden Wirkstoff und/oder mindestens einen Kühlwirkstoff und/oder mindestens einen pH-Regulierungswirkstoff und/oder mindestens einen Nährwirkstoff und/oder mindestens einen auffrischenden Wirkstoff aufweist.

3. Intimhautpflegeschleier (1) gemäß Anspruch 2, wobei das Zusatzgemisch den mindestens einen Hautweichmachwirkstoff in der Form eines okkludierenden Fettes aufweist.

4. Intimhautpflegeschleier (1) gemäß einem der Ansprüche 2 bis 3, wobei das Zusatzgemisch den mindestens einen Hautweichmachwirkstoff in der Form von feuchtigkeitsbindenden Substanzen aufweist.

5. Intimhautpflegeschleier (1) gemäß einem der Ansprüche 2 bis 4, wobei das Zusatzgemisch den mindestens einen Kühlwirkstoff aufweist.

6. Intimhautpflegeschleier (1) gemäß einem der Ansprüche 1 bis 4, wobei die Trägersubstanz mindestens eines der folgenden umfasst: Propylenglykol, Wasser, Dimeticon, Öl, Petrolatum und Alkohol.

7. Intimhautpflegeschleier (1) gemäß einem der Ansprüche 1 bis 6, der ferner eine Hinterkante (12), die sich im Wesentlichen in der Querrichtung des Schleiers (1) erstreckt, an dem hinteren Ende (3) des Schleiers (1) aufweist, wobei die Hinterkante (12) die erste Seitenkante (6a) an einem ersten Hinterübergangspunkt (T2a) schneidet und die zweite Seitenkante (6b) an einem zweiten Hinterübergangspunkt (T2b) schneidet, wodurch sich die ersten und zweiten Seitenkanten (6a, 6b) zwischen jeweiligen der Vorderübergangspunkte (T1a, T1b) und jeweiligen der Hinterübergangspunkte (T2a, T2b) erstrecken und der Hauptabschnitt (7) sich in der Längsrichtung zwischen der Vorderquerlinie (5) und einer geraden Hinterquerlinie (13) zwischen den ersten und zweiten Hinterübergangspunkten (T2a, T2b) erstreckt, wobei die gerade Hinterquerlinie (13) zu der Längsmittellinie (A) senkrecht ist.

8. Intimhautpflegeschleier (1) gemäß einem der Ansprüche 1 bis 7, wobei der Hauptabschnitt eine Vorderquerbreite (W1) und eine Hinterquerbreite (W2) hat, wobei das Verhältnis W1/W2 1,1 bis 6,6, bevorzugt 1,5 bis 4,2, mehr bevorzugt 2,8 bis 3,5 ist und wobei die Vorderquerbreite (W1) 100 bis 240 mm, bevorzugt 140 bis 210 mm, mehr bevorzugt 155 bis 175 mm ist und die Hinterquerbreite (W2) 25 bis 95 mm, mehr bevorzugt 40 bis 80 mm, am meisten bevorzugt 55 bis 70 mm ist.

9. Intimhautpflegeschleier (1) gemäß einem der Ansprüche 1 bis 8, wobei das Verhältnis L1/L2 0,5 bis 1, bevorzugt 0,6 bis 0,9, mehr bevorzugt 0,75 bis 0,85 ist und wobei die Längslänge (L1) des Hauptabschnittes (7) bevorzugt 60 bis 170 mm ist und die Gesamtlängslänge (L2) des Schleiers bevorzugt 60 bis 220 mm, bevorzugt 80 bis 150 mm, mehr bevorzugt 90 bis 120 mm ist.

10. Intimhautpflegeschleier (1) gemäß einem der Ansprüche 1 bis 9, wobei der Hauptabschnitt (7) so verjüngt ist, dass eine gerade Linie (6') zwischen dem ersten Vorderübergangspunkt (T1a) und dem ersten Hinterübergangspunkt (T2a) bezüglich der Vorderquerlinie (5) in einem Winkel (v2) geneigt ist, der kleiner als 90°, bevorzugt 20° bis 82°, mehr bevorzugt 50° bis 80°, am meisten bevorzugt 55° bis 75° ist.

11. Intimhautpflegeschleier (1) gemäß einem der Ansprüche 1 bis 10, wobei die erste Seitenkante (6a) eine gekrümmte konkave Form zwischen dem ersten Vorderübergangspunkt (T1a) und dem ersten Hinterübergangspunkt (T2a) hat und die zweite Seitenkante (6b) eine gekrümmte konkave Form zwischen dem zweiten Vorderübergangspunkt (T1b) und dem zweiten Hinterübergangspunkt (T2b) hat und wobei der Hauptabschnitt (7) bevorzugt einen Vorderhauptabschnitt (7a), der zwischen der Vorderquerlinie (5) und einer geraden Zwischenquerlinie (16) liegt, und einen Hinterhauptabschnitt (7b) aufweist, der zwischen der Hinterquerlinie (13) und der Zwischenquerlinie (16) liegt, und wobei eine Längslänge des Vorderhauptabschnitts (7a) dieselbe ist wie eine Längslänge des Hinterhauptabschnitts (7b) und wobei die gekrümmten konkaven Formen der ersten und zweiten Seitenkanten (6a, 6b) jeweils eine Krümmung an jeweiligen Zwischenübergangspunkten (T3a) ändern, sodass sie jeweilige Krümmungsrichtungen annehmen, und in denselben jeweiligen Krümmungsrichtungen fortlaufen, wobei die jeweiligen Zwischenübergangspunkte (T3a) in dem Hinterhauptabschnitt (7b) zwischen der Zwischenquerlinie (16) und der Hinterquerlinie (13) liegen und der Hauptabschnitt bevorzugt eine Zwischenquerbreite (W3) von 65 bis 90 mm an der Zwischenquerlinie (16) hat.

12. Intimhautpflegeschleier (1) gemäß Anspruch 11, wobei die ersten und zweiten Seitenkanten (6a, 6b) eine Krümmung ändern, sodass eine gerade Linie (6") zwischen dem ersten Hinterübergangspunkt (T2a) und dem Zwischenübergangspunkt (T3a) bezüglich der Hinterquerlinie (13) in einem Winkel (α) geneigt ist, der größer ist als 90°, und eine gerade Linie (6"') zwischen dem ersten Vorderübergangspunkt (T1a) und dem Zwischenübergangspunkt (T3a) bezüglich der Vorderquerlinie (5) in einem Winkel (β) geneigt ist, der kleiner ist als 180° - (α).

13. Intimhautpflegeschleier (1) gemäß Anspruch 12, wobei der Winkel (α) kleiner ist als der Winkel (v1) zwischen der Hinterquerlinie (13) und der geraden Linie (6') zwischen dem ersten Hinterübergangspunkt (T2a) und dem ersten Vorderübergangspunkt (T1a) und der Winkel (β) kleiner ist als der Winkel (v2) zwischen der geraden Linie (6') und der Vorderquerlinie (5).

14. Intimhautpflegeschleier (1) gemäß einem der Ansprüche 1 bis 13, der eine Gurley-Steifigkeit von 5 bis 300 mgf, mehr bevorzugt von 5 bis 100 mgf hat.

15. Intimhautpflegeschleier (1) gemäß einem der Ansprüche 1 bis 14, wobei die raumerzeugende Schicht eine vereinigte Schicht (10) ist, die die Speicherschicht (17) und die Oberdecke (18) aufweist, und wobei die Speicherschicht (17) der raumerzeugenden Schicht (10) bevorzugt ein Basisgewicht von 15 bis 100 g/m² und eine Dicke von 0,5 bis 3,5 mm hat.

16. Intimhautpflegeschleier (1) gemäß einem der Ansprüche 1 bis 14, wobei die raumerzeugende Schicht eine Mehrlagenschicht (10') ist, die den Speicherabschnitt (17') und den Oberdeckenabschnitt (18') aufweist.

17. Intimhautpflegeschleier (1) gemäß Anspruch 3, wobei das okkludierende Fett Petrolatum umfasst.

18. Intimhautpflegeschleier (1) gemäß Anspruch 4, wobei die feuchtigkeitsbindenden Substanzen Pidolsäure, Urea, Milchsäure, Glycerin oder Propylenglykol umfassen.

19. Intimhautpflegeschleier (1) gemäß Anspruch 5, wobei der Kühlwirkstoff Ketale, Carboxamide, Cyclohexylderivate, Cyclohexanolderivate, Mentholderivate, Kampfer, Borneol, Eucalyptol, Methylsalicylat, Teebaumöl oder Eukalyptusöl umfasst.

## Revendications

1. Voile revitalisant pour peau intime (1), configuré pour être positionné dans un sous-vêtement de sorte qu'il recouvre un mont du pubis devant une commissure antérieure des lèvres d'une utilisatrice, sans recouvrir une quelconque partie d'une zone du vestibule vulvaire de l'utilisatrice, dans lequel
le voile est sensiblement non-absorbant, en étant configuré pour absorber 0,9 % en poids de solution de chlorure de sodium selon une quantité de 0 à 2 fois son propre poids, et
le voile (1) présente une ligne centrale longitudinale (A) s'étendant dans la direction longitudinale du voile (1), et une première extension dans la direction longitudinale, entre une extrémité avant (2) et une extrémité arrière (3) du voile, et une seconde extension dans une direction transversale perpendiculaire à la ligne centrale longitudinale (A), et
dans lequel le voile (1) comprend un bord avant (4) et des premier et second bords latéraux (6a, 6b), le bord avant (4) entrecoupe le premier bord latéral (6a) à un premier point de transition avant (T1a) et entrecoupe le second bord latéral (6b) à un second point de transition avant (T1b), le voile (1) comprend en outre une partie principale (7) ayant une longueur (L1) dans la direction longitudinale du voile, le voile (1) a une longueur longitudinale totale (L2), la longueur (L1) de la partie principale (7) étant au moins égale à 50 % de la longueur longitudinale totale (L2) du voile (1), la partie principale (7) étant délimitée par une ligne droite transversale avant (5) entre les premier et second points de transition avant (T1a, T1b) et par les premier et second bords latéraux (6a, 6b), et se rétrécit vers l'extrémité arrière (3) du voile, la partie principale (7) présentant une symétrie de type réflexion par rapport à la ligne centrale longitudinale (A),
dans lequel le voile (1) a un côté faisant face au vêtement (9) et un côté au contact de la peau (11), le côté faisant face au vêtement (9) et le côté au contact de la peau (11) font face à l'opposé l'un de l'autre, et le voile (1) comprend une couche faisant face au vêtement (8) disposée sur le côté faisant face au vêtement (9) du voile (1), et une couche de création d'espace (10, 10') disposée sur le côté au contact de la peau (11) du voile (1), la couche de création d'espace (10, 10') comprenant une couche surélevée (17) et une feuille supérieure (18), la couche surélevée (17) étant positionnée entre la couche faisant face au vêtement (8) et la feuille supérieure (18), ou une partie surélevée (17') et une partie de feuille supérieure (18'), la partie surélevée (17') étant positionnée entre la couche faisant face au vêtement (8) et la partie de feuille supérieure (18'), et
dans lequel le voile (1) comprend en outre une composition additive sous la forme de : (i) un agent de bien-être cutané ; ou (ii) l'agent de bien-être cutané et une substance de support supportant l'agent de bien-être cutané, et
dans lequel la composition additive comprenant l'agent de bien-être cutané est comprise dans la couche surélevée (17) ou la partie surélevée (17') de la couche de création d'espace (10, 10').

2. Voile revitalisant pour peau intime (1) selon la revendication 1, dans lequel la composition additive comprend au moins un agent adoucissant la peau, et/ou au moins un agent hydratant, et/ou au moins un agent rafraîchissant, et/ou au moins un agent de régulation du pH, et/ou au moins un agent nourrissant et/ou au moins un agent régénérant.

3. Voile revitalisant pour peau intime (1) selon la revendication 2, dans lequel la composition additive comprend le au moins un agent adoucissant la peau sous la forme d'une graisse d'occlusion.

4. Voile revitalisant pour peau intime (1) selon l'une quelconque des revendications 2 à 3, dans lequel la composition additive comprend le au moins un agent adoucissant la peau sous la forme de substances d'hydro-rétention.

5. Voile revitalisant pour peau intime (1) selon l'une quelconque des revendications 2 à 4, dans lequel la composition additive comprend l'au moins un agent rafraîchissant.

6. Voile revitalisant pour peau intime (1) selon l'une quelconque des revendications 1 à 4, dans lequel la substance de support comprend au moins l'un parmi : propylène glycol, eau, diméticone, huile, vaseline, et alcool.

7. Voile revitalisant pour peau intime (1) selon l'une quelconque des revendications 1 à 6, comprenant en outre un bord arrière (12) qui s'étend sensiblement dans la direction transversale du voile (1), à l'extrémité arrière (3) du voile (1), le bord arrière (12) entrecoupant le premier bord latéral (6a) à un premier point de transition arrière (T2a) et entrecoupant le second bord latéral (6b) à un second point de transition arrière (T2b), de sorte que les premier et second bords latéraux (6a, 6b) s'étendent entre des point respectifs des points de transition avant (T1a, T1b) et des points respectifs des points de transition arrière (T2a, T2b), et la partie principale (7) s'étend dans la direction longitudinale entre la ligne transversale avant (5) et une ligne droite transversale arrière (13) entre les premier et second points de transition arrière (T2a, T2b), la ligne droite transversale arrière (13) étant perpendiculaire à la ligne centrale longitudinale (A).

8. Voile revitalisant pour peau intime (1) selon l'une quelconque des revendications 1 à 7, dans lequel la partie principale a une largeur transversale avant (W1) et une largeur transversale arrière (W2), dans lequel le rapport W1/W2 est de 1,1 à 6,6, de préférence de 1,5 à 4,2, de manière plus préférée de 2,8 à 3,5, et dans lequel la largeur transversale avant (W1) est de 100 à 240 mm, de préférence de 140 à 210 mm, de manière plus préférée de 155 à 175 mm, et la largeur transversale arrière (W2) est de 25 à 95 mm, de manière plus préférée de 40 à 80 mm, de manière la plus préférée de 55 à 70 mm.

9. Voile revitalisant pour peau intime (1) selon l'une quelconque des revendications 1 à 8, dans lequel le rapport L1/L2 est de 0,5 à 1, de préférence de 0,6 à 0,9, de manière plus préférée de 0,75 à 0,85, et dans lequel la longueur longitudinale (L1) de la partie principale (7) est de préférence de 60 à 170 mm, et la longueur longitudinale totale (L2) du voile est de préférence de 60 à 220 mm, de préférence de 80 à 150 mm, de manière plus préférée de 90 à 120 mm.

10. Voile revitalisant pour peau intime (1) selon l'une quelconque des revendications 1 à 9, dans lequel la partie principale (7) est effilée de telle sorte qu'une ligne droite (6') entre le premier point de transition avant (T1a) et le premier point de transition arrière (T2a), est inclinée par rapport à la ligne transversale avant (5) à un angle (v2), qui est inférieur à 90°, de préférence de 20 à 82°, de manière plus préférée de 50 à 80°, de manière la plus préférée de 55 à 75°.

11. Voile revitalisant pour peau intime (1) selon l'une quelconque des revendications 1 à 10, dans lequel le premier bord latéral (6a) a une forme concave incurvée entre le premier point de transition avant (T1a) et les premiers points de transition arrière (T2a), et le second bord latéral (6b) a une forme concave incurvée entre le second point de transition avant (T1b) et les seconds points de transition arrière (T2b), et dans lequel la partie principale (7) comprend de préférence une partie principale avant (7a) située entre la ligne transversale avant (5) et une ligne droite transversale intermédiaire (16), et une partie principale arrière (7b) située entre la ligne transversale arrière (13) et la ligne transversale intermédiaire (16), et dans lequel une longueur longitudinale de la partie principale avant (7a) est la même qu'une longueur longitudinale de la partie principale arrière (7b), et dans lequel les formes concaves incurvées des premier et second bords latéraux (6a, 6b) changent chacune de courbure aux points de transition intermédiaires respectifs (T3a) pour adopter des directions de courbure respectives, et continuent de s'étendre dans les mêmes directions de courbure respectives, dans lequel les points de transition intermédiaires respectifs (T3a) sont situés dans la partie principale arrière (7b) entre la ligne transversale intermédiaire (16) et la ligne transversale arrière (13), et la partie principale a de préférence une largeur transversale intermédiaire (W3) de 65 à 90 mm au niveau de la ligne transversale intermédiaire (16).

12. Voile revitalisant pour peau intime (1) selon la revendication 11, dans lequel les premier et second bords latéraux (6a, 6b) changent de courbure de telle sorte qu'une ligne droite (6") entre le premier point de transition arrière (T2a) et le point de transition intermédiaire (T3a), est inclinée par rapport à la ligne transversale arrière (13) à un angle (α), qui est > 90°, et qu'une ligne droite (6"') entre le premier point de transition avant (T1a) et le point de transition intermédiaire (T3a) est inclinée par rapport à la ligne transversale avant (5) à un angle (β), qui est < 180°-(α).

13. Voile revitalisant pour peau intime (1) selon la revendication 12, dans lequel l'angle (α) est plus petit que l'angle (v1) entre la ligne transversale arrière (13) et la ligne droite (6') entre le premier point de transition arrière (T2a) et le premier point de transition avant (T1a), et l'angle (β) est plus petit que l'angle (v2) entre la ligne droite (6') et la ligne transversale avant (5).

14. Voile revitalisant pour peau intime (1) selon l'une quelconque des revendications 1 à 13, ayant une rigidité Gurley de 5 à 300 mgf, de manière plus préférée de 5 à 100 mgf.

15. Voile revitalisant pour peau intime (1) selon l'une quelconque des revendications 1 à 14, dans lequel la couche de création d'espace est une couche combinée (10) comprenant la couche surélevée (17) et la feuille supérieure (18), et dans lequel la couche surélevée (17) de la couche de création d'espace (10) a de préférence un poids de base de 15 à 100 g/m² et une épaisseur de 0,5 à 3,5 mm.

16. Voile revitalisant pour peau intime (1) selon l'une quelconque des revendications 1 à 14, dans lequel la couche de création d'espace est une couche multicouche (10') comprenant la partie surélevée (17') et la partie de feuille supérieure (18').

17. Voile revitalisant pour peau intime (1) selon la revendication 3, dans lequel la graisse d'occlusion comprend de la vaseline.

18. Voile revitalisant pour peau intime (1) selon la revendication 4, dans lequel les substances d'hydro-rétention comprennent de l'acide pidolique, urée, acide lactique, glycérol, ou propylène glycol.

19. Voile revitalisant pour peau intime (1) selon la revendication 5, dans lequel l'agent rafraîchissant comprend des cétals, carboxamides, dérivés de cyclohexyle, dérivés de cyclohexanol, dérivés de menthol, camphre, bornéol, eucalyptol, salicylate de méthyle, huile d'arbre à thé, ou huile d'eucalyptus.
